# FASCICULE DE BREVET EUROPEEN

(11) **EP 1 120 091 B1**
(45) Date de publication et mention de la délivrance du brevet: **24.09.2008**
(21) Numéro de dépôt: 01400202.6
(22) Date de dépôt: 25.01.2001
(51) Int. Cl.: A61B 19/02, B65D 43/02

(54) **Récipient pour déchets infectieux comprenant un couvercle à fermeture temporaire**
Behälter für infektösen Müll mit Verschlussdeckel zum zeitweiligen Verschliessen
Container for infectious waste with a closing lid for temporary sealing

(30) Priorité: 25.01.2000 FR 0000901
(43) Date de publication de la demande: 01.08.2001
(73) Titulaire: Plazur, 21160 Couchey (FR)
(72) Inventeur: Charles, Daniel, 21160 Couchey (FR)
(74) Mandataire: Honoré, Anne-Claire

(56) Documents cités:
- EP-A- 0 629 169
- DE-U- 9 317 231
- FR-A- 816 341
- US-A- 4 813 563

## Description

La présente invention concerne un récipient de collecte et de transport de produits infectieux ou toxiques selon le préambule de la revendication 1 et particulièrement destiné aux milieux hospitaliers et médicaux comprenant sur son couvercle supérieur ou sur l'une de ses parois un couvercle de remplissage à fermeture temporaire pour introduire les déchets aisément sans ouvrir le couvercle principal du récipient, et divulgué dans le EP 0 629 169.

Dans le domaine des récipients de collecte de produits infectieux destinés aux milieux hospitaliers et médicaux, on connaît bien des récipients comprenant un corps parallélépipédique ou cylindro-conique, muni d'un couvercle principal pourvu d'une gorge périphérique garnie d'un joint élastique et poisseux qui s'adapte sur le bord supérieur du corps pour assurer l'étanchéité de la fermeture et une ouverture circulaire en creux pratiquée dans le couvercle susceptible d'accueillir un couvercle circulaire dit de remplissage à fermeture temporaire.

Généralement, les couvercles circulaires de ces récipients permettent leur remplissage progressif en assurant une première position de fermeture temporaire puis, lorsque le récipient est rempli, une seconde position de fermeture définitive avantageusement étanche. A cet égard, la paroi périphérique extérieure du couvercle de remplissage et la paroi périphérique externe de l'ouverture en creux sont équipées d'un jeu de cames qui coopèrent pour le verrouillage du couvercle de remplissage par sa rotation dans l'ouverture en creux.

C'est, par exemple, le cas du récipient de collecte pour produits infectieux proposé dans le brevet européen EP 0 629 169 qui décrit des cames positionnées sur la paroi périphérique extérieure du couvercle et constituées de crochets flexibles qui coopèrent avec des cames de la paroi périphérique externe de l'ouverture, c'est-à-dire la paroi de l'ouverture en creux visible par l'utilisateur lorsque le couvercle n'y est pas introduit. Ces cames, disposées en rangées, s'étendent de façon périphérique, c'est-à-dire perpendiculairement à la paroi périphérique externe de l'ouverture, à différentes hauteurs par rapport à l'axe de rotation du couvercle. La fermeture définitive du couvercle circulaire est obtenue par la disposition particulière des cames à des hauteurs différentes ou par la forme des cames de la paroi périphérique extérieure du couvercle et/ou des cames de la paroi périphérique externe de l'ouverture qui comprennent des indentations empêchant tout mouvement dudit couvercle après sa fermeture.

Afin de fermer le couvercle dans l'ouverture, l'utilisateur doit, dans une première variante d'exécution, tourner le couvercle dans le sens des aiguilles d'une montre, opérer une première pression verticale sur le couvercle, puis opérer une rotation dans le sens inverse des aiguilles d'une montre pour que le couvercle soit en position de fermeture temporaire et, finalement, en exerçant une deuxième pression verticale sur ledit couvercle, ce dernier est définitivement fermé en principe d'une manière étanche. Selon une seconde variante d'exécution, les cames de la paroi périphérique extérieure du couvercle comprennent des indentations ; l'utilisateur doit alors tourner le couvercle dans le sens des aiguilles d'une montre en exerçant simultanément une pression verticale sur le couvercle jusqu'à ce que les crochets de la paroi périphérique extérieure du couvercle soient positionnées dans les dernières indentations des cames pour verrouiller définitivement le couvercle.

Ce type de moyens de verrouillage du couvercle circulaire dans l'ouverture en creux présentent l'inconvénient pour l'utilisateur d'opérer, aussi bien pour l'ouverture que la fermeture, une succession de mouvements qui nécessitent l'utilisation des deux mains ce qui est particulièrement délicat lorsque l'utilisateur veut introduire des déchets qu'il tient déjà dans l'une de ses mains. Par ailleurs, les récipients ainsi que leurs couvercles circulaires sont classiquement en matière plastique de sorte qu'ils se déforment, rendant l'introduction des crochets flexibles dans les cames particulièrement difficile. De plus, de tels couvercles circulaires, qui sont habituellement munis d'une gorge périphérique garnie d'un joint poisseux et élastique coopérant avec une bride annulaire positionnée à la périphérie de l'ouverture en creux pour assurer l'étanchéité de la fermeture définitive, ne permettent pas d'obtenir l'étanchéité de leur fermeture temporaire occasionnant ainsi une insécurité pour les utilisateurs jusqu'à leur fermeture définitive.

L'un des buts de l'invention est donc de palier ces inconvénients en proposant un récipient de collecte de produits infectieux comprenant un couvercle de remplissage dont la paroi périphérique extérieure comprend des moyens coopérant avec la paroi périphérique externe d'une ouverture en creux afin de positionner aisément ledit couvercle dans l'ouverture et de le verrouiller temporairement, puis définitivement, par une simple rotation autour de son axe de symétrie central en assurant l'étanchéité des fermetures tant temporaire que définitive.

A cet égard et conformément à l'invention, le récipient de collecte et de transport de produits infectieux ou toxiques particulièrement destinés aux milieux hospitaliers et médicaux comprenant un corps parallélépipédique ou cylindro-conique fermé par un couvercle principal pourvu d'une gorge périphérique garnie d'un joint élastique qui s'adapte sur le bord supérieur du corps pour assurer l'étanchéité de la fermeture et une ouverture en creux pratiquée dans le couvercle susceptible d'accueillir un couvercle de remplissage dont l'extrémité supérieure comprend des moyens de préhension, la paroi périphérique extérieure du couvercle de remplissage et la paroi périphérique externe de l'ouverture en creux possédant des moyens coopérant pour le verrouillage par rotation autour de son axe de symétrie centrale du couvercle de remplissage dans l'ouverture ; ledit récipient est d'abord remarquable en ce que l'ouverture en creux et/ou le couvercle de remplissage sont des troncs de cônes de section décroissante depuis le couvercle principal jusqu'à l'intérieur du corps, homothétiques ou non, de sorte que ledit couvercle de remplissage soit apte à s'ajuster dans ladite ouverture en creux et en ce que la paroi périphérique extérieure du couvercle de remplissage ou la paroi extérieure de l'ouverture en creux comprennent des moyens d'étanchéité coopérant avec des moyens complémentaires disposés respectivement sur la paroi extérieure de l'ouverture en creux ou sur la paroi extérieure du couvercle de remplissage pour assurer une étanchéité aussi bien lors de la fermeture provisoire que lors de la fermeture définitive du couvercle de remplissage.

Selon une caractéristique essentielle du récipient conforme à l'invention, les moyens de verrouillage sont constitués d'au moins une patte ou une came hélicoïdale globalement perpendiculaire à l'axe de rotation A du couvercle de remplissage, solidaires de la paroi périphérique extérieure ou du fond dudit couvercle de remplissage coopérant avec une came hélicoïdale s'étendant à l'extrémité inférieure de l'ouverture en creux dans une direction globalement parallèle à l'axe de rotation A du couvercle, l'ouverture en creux comprenant le long de sa paroi externe au moins une percée verticale pour laisser le passage de ladite patte ou de ladite came hélicoïdale du couvercle qui peut ainsi venir au niveau de l'entrée de la came de l'ouverture en creux.

On comprend bien que la fermeture du couvercle de remplissage s'effectue simplement par sa rotation dans le sens des aiguilles d'une montre ou dans le sens inverse, c'est-à-dire dans le sens trigonométrique, suivant l'orientation des cames hélicoïdales, la fermeture temporaire étant obtenue par une rotation suivant un angle déterminé, notamment, par le nombre et par l'inclinaison des cames et la fermeture définitive étant obtenue par une rotation plus importante dudit couvercle de remplissage. Par ailleurs, l'étanchéité est assurée dés la fermeture temporaire du couvercle par la conicité de l'ouverture en creux et/ou du couvercle de remplissage.

D'autres avantages et caractéristiques ressortiront mieux de la description qui va suivre, de plusieurs variantes d'exécution, données à titre d'exemples non limitatifs, du récipient conforme à l'invention, en référence aux dessins annexés sur lesquels :
- la figure 1 est une vue en perspective éclatée du récipient et de son couvercle de remplissage conforme à l'invention,
- la figure 2 est une vue partielle en coupe diamétrale du couvercle de remplissage et du couvercle principal comprenant une ouverture en creux conforme à l'invention,
- la figure 3 est une vue de côté du couvercle principal comprenant l'ouverture en creux muni du couvercle de remplissage conforme à l'invention en position de fermeture temporaire,
- la figure 4 est une vue de dessus du couvercle principal montrant l'ouverture en creux conforme à l'invention,
- la figure 5 est une vue partielle en coupe de la collerette du couvercle de remplissage en position fermée dans l'ouverture en creux du couvercle principal,
- la figure 6 est une vue partielle en coupe selon l'axe VI-VI' de la figure 5,
- la figure 7 est une vue de côté du couvercle principal comprenant l'ouverture en creux muni du couvercle de remplissage en position de fermeture temporaire montrant une variante d'exécution des cames hélicoïdales conformes à l'invention,
- la figure 8 est une vue partielle en coupe diamétrale d'une variante d'exécution du couvercle de remplissage et du couvercle principal conforme à l'invention.

On décrira, dans cet exemple non limitatif, un récipient globalement parallélépipédique destiné à la collecte de produits infectieux comprenant un couvercle de remplissage de fermeture temporaire conforme à l'invention.

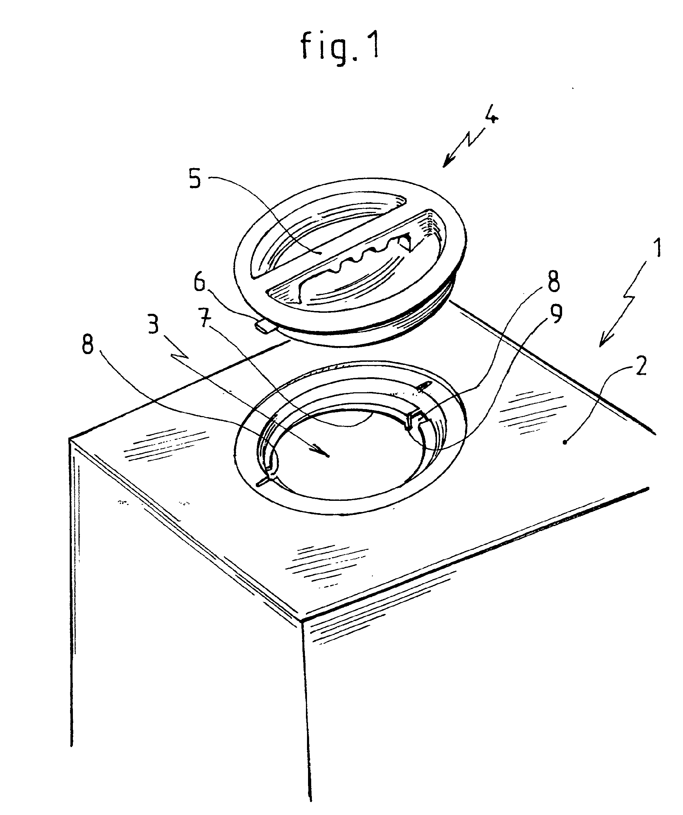

Le récipient, en référence à la figure 1, est constitué d'un corps 1 globalement parallélépipédique, classiquement posé verticalement sur le sol, et d'un couvercle principal 2 de forme globalement rectangulaire classiquement pourvu d'une gorge périphérique garnie d'un joint élastique et poisseux qui s'adapte sur le bord supérieur du corps 1 pour assurer l'étanchéité de sa fermeture. Le couvercle principal 2 comprend dans sa partie centrale une ouverture en creux 3 globalement circulaire apte à accueillir un couvercle 4 dit de remplissage, la paroi périphérique extérieure dudit couvercle de remplissage 4 et la paroi périphérique externe de l'ouverture en creux 3 possédant des moyens qui permettent d'assurer la fermeture provisoire puis la fermeture définitive dudit couvercle 4 comme on le verra plus loin.

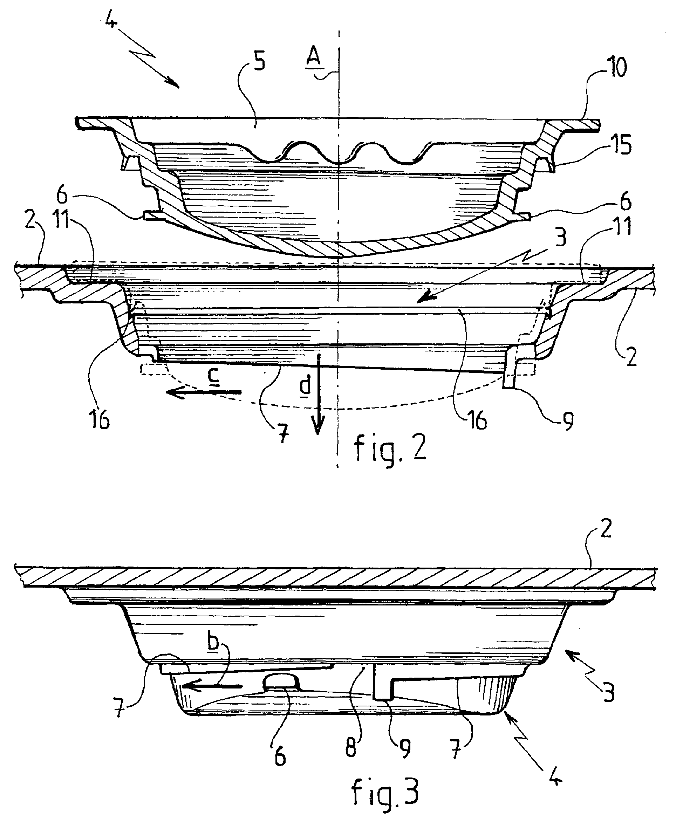

Le couvercle de remplissage 4, en référence aux figures 1 et 2, comprend des moyens de préhension constitués par une poignée 5 avantageusement ergonomique s'étendant diamétralement entre les bords dudit couvercle 4. Par ailleurs, la paroi périphérique extérieure du couvercle de remplissage 4 comprend à son extrémité inférieure, c'est-à-dire à son extrémité comprise dans le volume du récipient lorsque ledit couvercle 4 est introduit dans l'ouverture en creux 3, deux pattes 6 perpendiculaires à l'axe de rotation A dudit couvercle de remplissage 4. Les pattes 6 sont avantageusement disposées de part et d'autre de l'axe de rotation A dudit couvercle 4 pour répartir les efforts comme on le verra plus loin. Lors de la fermeture du couvercle de remplissage 4 dans l'ouverture en creux 3, les pattes 6 coopèrent respectivement avec une came 7 hélicoïdale s'étendant à l'extrémité inférieure de l'ouverture 3 dans une direction globalement parallèle à l'axe de rotation A du couvercle 4. De plus, l'ouverture en creux 3 comprend le long de sa paroi périphérique externe, et plus précisément à son extrémité inférieure, deux percées 8 pour laisser le passage desdites pattes 6 pour que ces dernières soient au niveau des cames 7 après l'introduction du couvercle 4 dans l'ouverture 3.

Il va de soi que la paroi périphérique extérieure du couvercle de remplissage 4 peut ne comprendre qu'une patte 6 positionnée à son extrémité inférieure ou solidaire du fond dudit couvercle 4 et s'étendant globalement perpendiculairement à l'axe de rotation A. Par ailleurs, la ou les pattes 6 peuvent avantageusement être substituées par une ou plusieurs cames hélicoïdales solidaires de la paroi périphérique extérieure du couvercle 4 coopérant avec la came hélicoïdale 7 de la paroi périphérique externe de l'ouverture en creux 3 afin que les moyens de fermeture puissent résister à des efforts de traction importants notamment lorsque les récipients pleins et définitivement fermés sont soulevés par la poignée 5 du couvercle de remplissage 4.

En référence à la figure 2, l'ouverture en creux 3 est avantageusement un tronc de cône de section décroissante depuis le couvercle principal 2 jusqu'à l'intérieur dudit corps 1, c'est-à-dire une section décroissante de haut en bas, et le couvercle de remplissage 4 présente une section en tronc de cône homothétique ou non à l'ouverture en creux 3 de dimension apte à s'y ajuster. Une telle configuration en tronc de cône permet un autocentrage du couvercle de remplissage 4 dans l'ouverture en creux 3 facilitant ainsi son introduction comme on le verra un peu plus loin.

Par ailleurs, les cames 7 qui s'étendent entre deux percées 8 consécutives croissent, en référence aux figures 2 et 3, progressivement en hauteur dans le sens des aiguilles d'une montre, c'est-à-dire de gauche à droite pour la came 7 représentée sur la figure 2. Lesdites cames 7 se terminent avantageusement par une butée 9 de telle sorte qu'après l'introduction du couvercle de remplissage 4, c'est-à-dire après l'introduction des pattes 6 dans les percées 8, l'utilisateur ne puisse tourner le couvercle de remplissage 4 que dans le sens des aiguilles d'une montre, comme l'indique la flèche b sur la figure 3, pour le fermer.

Il va de soi que les cames 7 qui s'étendent entre deux percées 8 consécutives peuvent croître progressivement en hauteur dans le sens trigonométrique, c'est-à-dire dans le sens inverse des aiguilles d'une montre, de sorte que la fermeture du couvercle 4 s'opère alors par sa rotation dans le sens trigonométrique. De plus, il est bien évident que le nombre de cames 7 et leurs pentes respectives dépendent du nombre de pattes 6 du couvercle de remplissage.

En référence à la figure 2, le couvercle de remplissage 4 comprend, par ailleurs, une collerette périphérique circulaire 10 destinée à prendre appui dans une feuillure 11 de forme correspondante entourant l'ouverture en creux 3.

Il va de soi que la collerette 10 du couvercle 4 peut prendre appui directement sur le couvercle principal 2 du récipient sans pour autant nuire à l'efficacité de la fermeture.

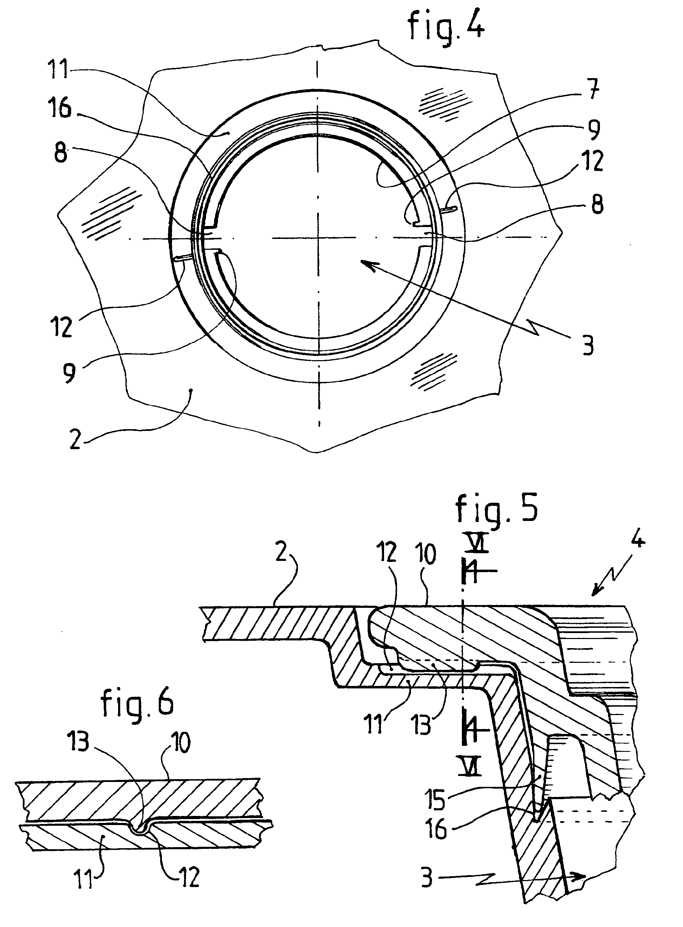

La feuillure 11 entourant l'ouverture en creux 3, en référence à la figure 4, comprend des excavations 12 diamétralement opposées coopérant avec des ergots 13 correspondants et complémentaires de la face interne de la collerette 10, c'est-à-dire de la face en regard de ladite feuillure 11 lors de la fermeture du couvercle de remplissage, représentés sur les figures 5 et 6. Les excavations 12 consistent en des rainures s'étendant radialement par rapport à l'axe de rotation A du couvercle de remplissage 4 et les ergots 13 présentent une forme complémentaire de telle sorte que, lors de la fermeture du couvercle 4, les ergots 13 soient positionnés dans les excavations 12 empêchant ainsi toute rotation dudit couvercle 4 qui est alors en position de fermeture définitive.

Selon une autre variante d'exécution non représentée sur les figures, La feuillure 11 entourant l'ouverture en creux 3 comprend des ergots 13 diamétralement opposées coopérant avec des excavations 12 correspondantes de la face interne de la collerette 10. De la même manière que précédemment, les excavations 12 consistent alors en des rainures s'étendant radialement par rapport à l'axe de rotation A du couvercle de remplissage 4 et les ergots 13 présentent une forme complémentaire.

Il va de soi que les excavations 12 et les ergots 13 peuvent avoir une position et une forme quelconque telle que, par exemple, une excavation 12 circulaire coopérant avec un ergot 13 en forme de demi-sphère. De plus, la collerette 10 et le couvercle principal 2 ou la feuillure 11 peuvent comprendre d'autres types de moyens coopérant pour la fermeture définitive du couvercle de remplissage 4 dans l'ouverture en creux 3, connus par l'homme du métier.

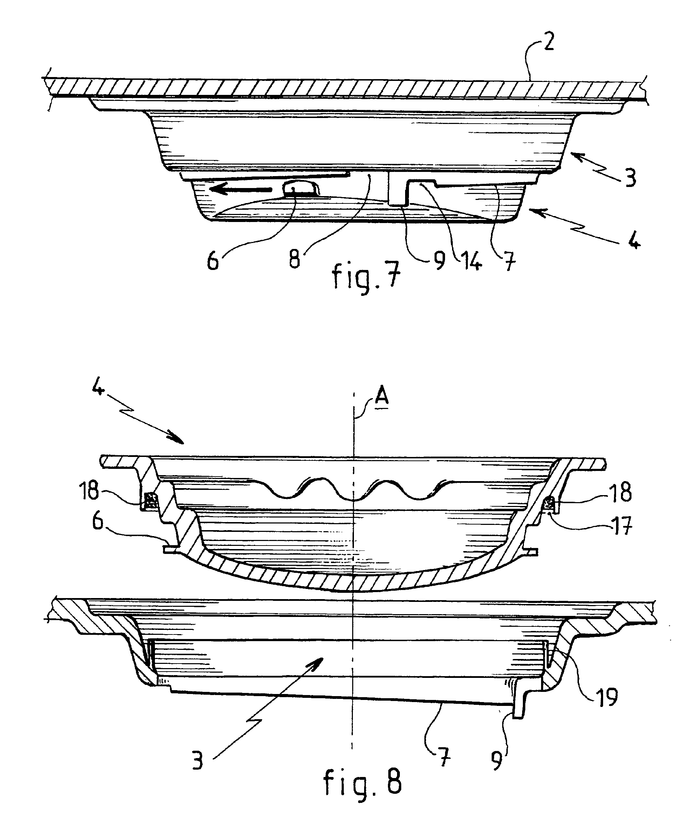

Selon une autre variante d'exécution du récipient conforme à l'invention, en référence à la figure 7, les cames 7 qui s'étendent entre deux percées 8 consécutives de la paroi périphérique externe de l'ouverture en creux 3 comprennent à leur extrémité respective, avant la butée 9 qui marque la fin de la came 7, une indentation 14 dans laquelle vient se loger la patte 6 du couvercle de remplissage 4 afin de bloquer définitivement la rotation dudit couvercle 4.

Afin de réaliser l'étanchéité de la fermeture temporaire ou définitive, la paroi périphérique externe du couvercle de remplissage 4 comprend, par ailleurs, en référence à la figure 2, une jupe périphérique 15 avantageusement déformable qui s'étend de haut en bas globalement parallèlement à l'axe de rotation A du couvercle de remplissage 4 en s'évasant légèrement vers l'extérieur, c'est-à-dire en formant un angle d'environ 5° avec l'axe de rotation A, de telle sorte que, lors de la fermeture dudit couvercle 4, elle prenne appui sur la paroi périphérique externe de l'ouverture en creux 3 assurant ainsi l'étanchéité de la fermeture. On notera que plus la fermeture est complète, c'est-à-dire plus la rotation du couvercle 4 est importante, plus la paroi périphérique extérieure du couvercle de remplissage 4 se rapproche de la paroi périphérique externe de l'ouverture en creux 3 du fait de leur conicité de sorte que la jupe 15 est comprimée avec de plus en plus de force sur la paroi périphérique externe de l'ouverture en creux 3 jusqu'à la fermeture définitive du couvercle 4 où l'étanchéité est alors parfaitement assurée.

Il est bien évident que la jupe 15 peut s'étendre en formant un angle quelconque avec l'axe de rotation A du couvercle ; toutefois, on a constaté une meilleure étanchéité de la fermeture temporaire ou définitive du couvercle 4 avec une jupe déformable 15 légèrement évasée s'étendant de haut en bas en format un angle compris entre 5° et 30° avec l'axe de rotation A dudit couvercle 4.

L'ouverture en creux 3 comprend avantageusement sur sa paroi périphérique externe une gorge annulaire 16 de section globalement en V orientée vers le haut, c'est-à-dire ouverte vers le haut, et apte à accueillir, lors de la fermeture du couvercle de remplissage 4 l'extrémité libre de la jupe périphérique 15 dudit couvercle 4 améliorant encore l'étanchéité de la fermeture.

Il va de soi que la gorge annulaire 16 peut avoir une section quelconque telle que, par exemple, en forme de U.

Selon une dernière variante d'exécution du récipient conforme à l'invention, en référence à la figure 8, la paroi périphérique extérieure du couvercle de remplissage 4 comprend une gorge périphérique 17, s'étendant globalement parallèlement à l'axe de rotation A dudit couvercle 4, garnie d'un joint élastique et poisseux 18. Ledit joint 18 coopère avec une bride annulaire 19 de la paroi périphérique de l'ouverture en creux 3 qui s'étend de bas en haut globalement parallèlement à l'axe de rotation A du couvercle de remplissage 4 afin d'assurer l'étanchéité de la fermeture.

On décrira maintenant le fonctionnement de la charnière à verrouillage en référence aux figures 1, 2, 3, 5 et 6.

Le couvercle de remplissage 4, préalablement ouvert pour permettre aux utilisateurs de jeter à travers l'ouverture en creux 3 des déchets tels que des seringues, par exemple, dans le corps 1, est introduit dans ladite ouverture en creux 3 en référence aux figures 1 et 2. L'utilisateur qui s'est saisi du couvercle 4 par sa poignée 5 opère alors une première rotation dans le sens des aiguilles d'une montre ou dans le sens trigonométrique jusqu'à ce que les pattes 6 soient engagées dans les percées 8, l'engagement desdites pattes 6 étant facilité par la conicité de l'ouverture en creux 3 et du couvercle de remplissage 4. Après l'engagement des pattes 6 dans les percées 8, l'utilisateur est alors apte à opérer la fermeture provisoire du couvercle de remplissage 4 représenté en traits pointillés sur la figure 2.

Les butées 9 des cames 7 empêchant toute rotation dans le sens inverse des aiguilles d'une montre, l'utilisateur ne peut tourner le couvercle 4 que dans le sens des aiguilles d'une montre comme l'indique la flèche c de la figure 2. Les pattes 6 prennent alors appui sur les cames 7 entraînant le couvercle de remplissage 4 en translation vers l'intérieur de l'ouverture en creux 3 comme l'indique la flèche d de sorte que la jupe déformable 15 entre en contact avec la paroi périphérique externe de l'ouverture en creux 3 assurant ainsi l'étanchéité de la fermeture. Par ailleurs, la collerette 10 du couvercle de remplissage 4 prend également appui dans la feuillure 11 et plus la rotation dudit couvercle 4 est importante, plus la pression de la collerette 10 exercée sur la feuillure 11 et la pression de la jupe déformable 15 sur la paroi périphérique externe de l'ouverture en creux 3 sont grandes, de sorte qu'une résistance au mouvement de rotation se crée aux alentours d'un quart de tour, c'est-à-dire pour une rotation de 90° dans le sens des aiguilles d'une montre indiquant à l'utilisateur que le couvercle 4 est en position de fermeture temporaire étanche. Le couvercle de remplissage 4 est alors en position de fermeture temporaire et l'utilisateur peut aisément réouvrir ledit couvercle 4 pour jeter de nouveaux déchets en opérant une simple rotation d'un quart de tour dans le sens inverse des aiguilles d'une montre.

Afin d'opérer une fermeture définitive du récipient, le couvercle de remplissage 4 est introduit dans l'ouverture en creux 3 comme précédemment, puis l'utilisateur effectue une rotation dudit couvercle 4 d'environ 180° dans le sens des aiguilles d'une montre jusqu'à ce que les ergots 13 de la collerette 10, représentés sur les figures 5 et 6, se logent dans les excavations 12 correspondantes de la feuillure 11. La pression exercée par la collerette 10 sur la feuillure 11 ainsi que les ergots 13 positionnés dans les excavations 12 empêchent alors l'ouverture du couvercle de remplissage 4 par l'utilisateur. Par ailleurs, la jupe déformable 15 entrée en contact avec la paroi périphérique externe de l'ouverture en creux 3 après l'introduction du couvercle 4 dans ladite ouverture en creux 3, est comprimée, lors de la rotation du couvercle 4, sur cette dernière jusqu'à ce que son extrémité libre prenne appui dans la gorge annulaire 16 de l'ouverture en creux 3 assurant ainsi une parfaite étanchéité de la fermeture.

Afin de réaliser l'étanchéité de la fermeture temporaire du couvercle de remplissage 4, il va de soi que seule l'ouverture en creux 3 peut être un tronc de cône de section décroissante depuis le couvercle principal 2 jusqu'à l'intérieur du corps 1, le couvercle circulaire étant globalement cylindrique, ou que seul le couvercle de remplissage 4 peut présenter une section en tronc de cône de dimension apte à s'ajuster dans une ouverture en creux 3 globalement cylindrique, sans pour autant sortir du cadre de l'invention.

Il est bien évident que le récipient, le couvercle principal 2 et le couvercle de remplissage 4 sont avantageusement obtenus dans une matière plastique rigide ou semi-rigide pour, notamment, réduire les coûts de fabrication de ces récipients et faciliter leur destruction ; toutefois, les différents éléments du récipient peuvent être obtenus dans n'importe quel type de matériau rigide ou semi-rigide sans sortir du cadre de l'invention.

Par ailleurs, l'ouverture en creux 3 peut être pratiquée dans l'une des parois du corps parallélépipédique 1 du récipient en fonction de son utilisation finale.

Enfin, il va de soi que le couvercle de remplissage du récipient conforme à l'invention peut être adapté à tous les types de containers et analogues et que les exemples que l'on vient de donner ne sont que des illustrations particulières en aucun cas limitatives des domaines d'application de l'invention.

## Revendications

1. Récipient de collecte et de transport de produits infectieux ou toxiques particulièrement destinés aux milieux hospitaliers et médicaux comprenant un corps (1) parallélépipédique ou cylindro-conique fermé par un couvercle principal (2) pourvu d'une gorge périphérique garnie d'un joint élastique qui s'adapte sur le bord supérieur du corps (1) pour assurer l'étanchéité de la fermeture et une ouverture en creux (3) pratiquée dans le couvercle principal (2) susceptible d'accueillir un couvercle de remplissage (4) comprenant des moyens de préhension (5), la paroi périphérique extérieure du couvercle de remplissage (4) et la paroi périphérique externe de l'ouverture en creux (3) possédant des moyens coopérant pour le verrouillage par rotation autour de son axe A de symétrie centrale dudit couvercle de remplissage (4) dans l'ouverture (3), **caractérisé en ce que** l'ouverture en creux (3) et/ou le couvercle de remplissage (4) sont des troncs de cônes de section décroissante depuis le couvercle principal (2) jusqu'à l'intérieur du corps (1), homothétiques ou non, de sorte que ledit couvercle de remplissage (4) soit apte à s'ajuster dans ladite ouverture en creux (3) et **en ce que** la paroi périphérique extérieure du couvercle de remplissage (4) ou la paroi extérieure de l'ouverture en creux (3) comprennent des moyens d'étanchéité coopérant avec des moyens complémentaires disposés respectivement sur la paroi extérieure de l'ouverture en creux (3) ou sur la paroi extérieure du couvercle de remplissage (4) pour assurer une étanchéité aussi bien lors de la fermeture provisoire que lors de la fermeture définitive du couvercle de remplissage (4).

2. Récipient selon la revendication précédente **caractérisé en ce que** les moyens de verrouillage sont constitués d'au moins une patte ou une came hélicoïdale (6) globalement perpendiculaire à l'axe de rotation A du couvercle de remplissage (4), solidaires de la paroi périphérique extérieure ou du fond dudit couvercle de remplissage (4) coopérant avec une came hélicoïdale (7) s'étendant à l'extrémité inférieure de l'ouverture en creux (3) dans une direction globalement parallèle à l'axe de rotation A du couvercle (4), l'ouverture en creux (3) comprenant le long de sa paroi externe au moins une percée (8) verticale pour laisser le passage de ladite patte ou de ladite came hélicoïdale (6) du couvercle (4) qui peut ainsi venir au niveau de l'entrée de la came (7) de l'ouverture en creux (3).

3. Récipient selon la revendication 2 **caractérisé en ce que** les cames (7) s'étendent entre deux percées (8) consécutives en croissant progressivement en hauteur dans le sens des aiguilles d'une montre ou inversement dans l'autre sens et se terminent par une butée (9) de sorte qu'après l'introduction du couvercle de remplissage (4), c'est-à-dire après l'introduction des pattes (6) dans lesdites percées (8), l'utilisateur ne puisse tourner le couvercle de remplissage (4) que dans le sens des aiguilles d'une montre, ou respectivement dans l'autre sens, pour fermer ledit couvercle (4).

4. Récipient selon l'une quelconque des revendications 1 à 3 **caractérisé en ce que** le couvercle de remplissage (4) comprend sur sa paroi périphérique extérieure une jupe périphérique (15) déformable qui prend appui sur la paroi périphérique externe de l'ouverture en creux (3) lors de la fermeture dudit couvercle (4) pour assurer l'étanchéité de la fermeture.

5. Récipient selon la revendication 4 **caractérisé en ce que** l'ouverture en creux (3) comprend sur sa paroi périphérique externe une gorge annulaire (16) ouverte vers le haut apte à accueillir, lors de la fermeture du couvercle de remplissage (4) l'extrémité libre de la jupe périphérique (15).

6. Récipient selon la revendication 5 **caractérisé en ce que** la gorge annulaire (16) présente une section globalement en forme de V.

7. Récipient selon l'une quelconque des revendications 1 à 3 **caractérisé en ce que** le couvercle de remplissage (4) comprend sur sa paroi périphérique extérieure une gorge périphérique (16) garnie d'un joint élastique et poisseux (17) qui coopère avec une bride annulaire (18) de la paroi périphérique externe de l'ouverture en creux (3) lors de la fermeture dudit couvercle (4) pour assurer l'étanchéité de la fermeture.

8. Récipient selon l'une quelconque des revendications précédentes **caractérisé en ce que** le couvercle de remplissage (4) comprend une collerette périphérique circulaire (10) destinée à prendre appui sur le couvercle principal (2) ou dans une feuillure (11) de forme correspondante entourant l'ouverture en creux (3) et **en ce que** la collerette périphérique (10) et le couvercle principal (2) ou la feuillure (11) comprend des moyens coopérant pour la fermeture définitive du couvercle de remplissage (4) dans l'ouverture en creux (3).

9. Récipient selon la revendication 8 **caractérisé en ce que** la face interne de la collerette (10), c'est-à-dire la face en regard de la feuillure (11) ou du couvercle principal (2) lors de la fermeture du couvercle de remplissage (4), comprend au moins un ergot (13) coopérant avec au moins une excavation (12) correspondante de la feuillure (11) ou du couvercle principal (2) pour assurer la fermeture définitive du couvercle de remplissage (4) dans l'ouverture en creux (3).

10. Récipient selon la revendication 9 **caractérisé en ce que** l'excavation (12) consiste en une rainure s'étendant radialement par rapport à l'axe de rotation A du couvercle de remplissage (4).

## Claims

1. Container for the collection and transport of infectious or toxic products particularly intended for hospital and medical environments comprising a parallelepipedic or cylindro-conical body (1) closed by a main lid (2) provided with a peripheral groove lined with an elastic seal that is adapted to the upper edge of the corps (1) in order to provide the seal for the closing and a hollow opening (3) made in the main lid (2) able to accommodate a filling lid (4) comprising gripping means (5), the exterior peripheral wall of the filling lid (4) and the external peripheral wall of the hollow opening (3) having means cooperating for the locking via rotation around its axis A of central symmetry of said filling lid (4) in the opening (3), **characterised in that** the hollow opening (3) and/or the filling lid (4) are frustums of cones that reduce down from the main lid (2) to the interior of the body (1), homothetic or not, in such a way that said filling lid (4) is able to be adjusted in said hollow opening (3) and **in that** the exterior peripheral wall of the filling lid (4) or the exterior wall of the hollow opening (3) include means of sealing cooperating with additional means arranged respectively on the exterior wall of the hollow opening (3) or on the exterior wall of the filling lid (4) in order to provide a seal that is as good during the temporary closing as during the definitive closing of the filling lid (4).

2. Container as set forth in the preceding claim **characterised in that** the means of locking are comprised of at least one tab or one helicoidal cam (6) globally perpendicular to the axis of rotation A of the filling lid (4), attached to the exterior peripheral wall or to the bottom of said filling lid (4) cooperating with a helicoidal cam (7) extending to the lower end of the hollow opening (3) in a direction that is globally parallel to the axis of rotation A of the lid (4), the hollow opening (3) comprising along its external wall at least one vertical aperture (8) to allow for the passage of said tab or of said helicoidal cam (6) of the lid (4) which can as such come to the level of the entry of the cam (7) of the hollow opening (3).

3. Container as set forth in claim 2 **characterised in that** the cams (7) extend between two consecutive apertures (8) increasing progressively in height in a clockwise direction or inversely in the other direction and ending by a stop (9) in such a way that after the introduction of the filling lid (4), i.e. after the introduction of the tabs (6) in said apertures (8), the user can turn the filling lid (4) only in a clockwise direction, or respectively in the other direction, in order to close said lid (4).

4. Container as set forth in any one of claims 1 to 3 **characterised in that** the filling lid (4) comprises on its exterior peripheral wall a deformable peripheral skirt (15) that takes support on the external peripheral wall of the hollow opening (3) during the closing of said lid (4) in order to provide the seal of the closing.

5. Container as set forth in claim 4 **characterised in that** the hollow opening (3) comprises on its external peripheral wall an annular groove (16) open upwards able to accommodate, during the closing of the filling lid (4) the free end of the peripheral skirt (15).

6. Container as set forth in claim 5 **characterised in that** the annular groove (16) presents a section that is globally in the form of a V.

7. Container as set forth in any one of claims 1 to 3 **characterised in that** the filling lid (4) comprises on its exterior peripheral wall a peripheral groove (16) lined with an elastic and tacky seal (17) that cooperates with an annular flange (18) of the external peripheral wall of the hollow opening (3) during the closing of said lid (4) in order to provide the seal of the closing.

8. Container as set forth in any one of the preceding claims **characterised in that** the filling lid (4) comprises a circular peripheral collar (10) intended to take support on the main lid (2) or in a fillister (11) of corresponding form surrounding the hollow opening (3) and **in that** the peripheral collar (10) and the main lid (2) or the fillister (11) comprise means cooperating to definitively close the filling lid (4) in the hollow opening (3).

9. Container as set forth in claim 8 **characterised in that** the internal face of the collar (10), i.e. the face across from the fillister (11) or from the main lid (2) during the closing of the filling lid (4), comprises at least one lug (13) cooperating with at least one corresponding excavation (12) of the fillister (11) or of the main lid (2) in order to provide the definitive closing of the filling lid (4) in the hollow opening (3).

10. Container as set forth in claim 9 **characterised in that** the excavation (12) consists of a groove extending radially in relation to the axis of rotation A of the filling lid (4).

## Patentansprüche

1. Behälter zum Sammeln und Befördern von spezifischen infektiösen oder toxischen Produkten in Krankenhäusern bzw. medizinischen Einrichtungen, mit einem quaderförmigen oder zylindrisch-konischen Behälterkörper (1), der durch einen Hauptdeckel (2) verschlossen ist, der eine umlaufende Hohlkehle besitzt, in die eine elastische Dichtung eingesetzt ist, die zur Gewährleistung der Dichtheit mit dem oberen Rand des Behälterkörpers (2) zusammenpasst, sowie einer im Hauptdeckel (2) vorgesehenen vertieften Öffnung (3), in die ein Einfülldeckel (4) eingesetzt werden kann, der Greifmittel (5) besitzt, wobei an der umlaufenden Außenwand des Einfülldeckels (4) und an der umlaufenden Außenwand der vertieften Öffnung (3) jeweils Verriegelungsmittel vorgesehen sind, die zum Verschließen des Einfülldeckels (4) durch dessen Drehung um seine zentrale Symmetrieachse A in der Öffnung (3) zusammenwirken, **dadurch gekennzeichnet, dass** die vertiefte Öffnung (3) bzw. der Einfülldeckel (4) Kegelstumpfprofile besitzen, deren Durchmesser vom Hauptdeckel (2) zum Inneren des Behälters (1) hin linear oder nichtlinear abnimmt, so dass sich der Einfülldeckel (4) in die vertiefte Öffnung (3) einpasst, sowie **dadurch**, dass an der Außenwand des Einfülldeckels (4) bzw. der vertieften Öffnung (3) Dichtmittel vorgesehen sind, die mit entsprechenden komplementären Mitteln zusammenwirken, die jeweils an der Außenwand der vertieften Öffnung (3) bzw. des Einfülldeckels (4) angebracht sind, um sowohl beim zeitweiligen, als auch beim endgültigen Verschließen des Behälters mit dem Einfülldeckel (4) ein einwandfreies Dichtschließen zu gewährleisten.

2. Behälter nach dem vorhergehenden Anspruch, **dadurch gekennzeichnet, dass** die Verriegelungsmittel aus mindestens einer Gewindelasche oder -nocke (6) bestehen, die weitgehend senkrecht zur Drehachse A des Einfülldeckels (4) ausgerichtet und fest mit der umlaufenden Außenwand oder dem Boden des Einfülldeckels (4) verbunden ist und mit einer Gewindenute (7) zusammenwirkt, die sich von der Innenkante der vertieften Öffnung (3) aus weitgehend senkrecht zur Drehachse A des Einfülldeckels (4) erstreckt, wobei die vertiefte Öffnung (3) auf ihrer Außenwand mindestens eine Aussparung (8) aufweist, durch die die Gewindelasche oder -nocke (6) des Einfülldeckels (4) hindurchpasst und somit mit der Gewindenute (7) der vertieften Öffnung (3) zusammenwirken kann.

3. Behälter nach Anspruch 2, **dadurch gekennzeichnet, dass** sich die Gewindenuten (7) zwischen zwei aufeinanderfolgenden Aussparungen (8) erstrecken, wobei ihre Höhe bei der Drehung im Uhrzeiger- oder Gegenuhrzeigersinn gleichmäßig zunimmt und mit einem Anschlag (9) endet, so dass der Anwender nach dem Einsetzen des Einfülldeckels (4), d.h. nach dem Durchführen von dessen Nocken (6) durch die Aussparungen (8) den Einfülldeckel (4) durch dessen Drehung im Uhrzeigersinn verschließen bzw. durch Drehung im Gegenuhrzeigersinn öffnen kann.

4. Behälter nach einem der Ansprüche 1 bis 3, **dadurch gekennzeichnet, dass** der Einfülldeckel (4) auf seiner umlaufenden Außenwand eine verformbare, umlaufende Schürze (15) aufweist, die nach dem Einsetzen des Einfülldeckels (4) an der umlaufenden Außenwand der vertieften Öffnung (3) anliegt, um ein einwandfreies Dichtschließen zu gewährleisten.

5. Behälter nach Anspruch 4, **dadurch gekennzeichnet, dass** an der umlaufenden Außenwand der vertieften Öffnung (3) eine nach oben offene, ringförmige Hohlkehle (16) vorgesehen ist, in die sich nach dem Einsetzen des Einfülldeckels (4) das freie Ende von dessen umlaufender Schürze (15) einpasst, um ein einwandfreies Dichtschließen zu gewährleisten.

6. Behälter nach Anspruch 5, **dadurch gekennzeichnet, dass** die ringförmige Hohlkehle (16) ei weitgehend V-förmiges Profil besitzt.

7. Behälter nach einem der Ansprüche 1 bis 3, **dadurch gekennzeichnet, dass** der Einfülldeckel (4) auf seiner umlaufenden Außenwand eine umlaufende Hohlkehle (16) aufweist, die mit einer elastischen und klebrigen Dichtung (17) bestückt ist, die mit einem ringförmigen Flansch (18) an der umlaufenden Außenwand der vertieften Öffnung (3) zusammenwirkt, um nach dem Einsetzen des Einfülldeckels (4) ein einwandfreies Dichtschließen zu gewährleisten.

8. Behälter nach einem der vorhergehenden Ansprüche, **dadurch gekennzeichnet, dass** der Einfülldeckel (4) einen umlaufenden kreisförmigen Kragenwulst (10) besitzt, der dazu dient, auf dem Hauptdeckel (2) aufzuliegen oder in eine um die vertiefte Öffnung (3) verlaufende Anschlagfuge (11) entsprechender Form zu passen, und dass der umlaufende kreisförmige Kragenwulst (10) und der Hauptdeckel (2) oder die Anschlagfuge (11) jeweils Mittel besitzen, die zum endgültigen Verschließen des Einfülldeckels (4) nach dessen Einsetzen in die vertiefte Öffnung (3) zusammenwirken.

9. Behälter nach Anspruch 8, **dadurch gekennzeichnet, dass** auf der Innenseite des Kragenwulstes (10), d.h. auf der beim Verschließen des Einfülldeckels (4) der Anschlagfuge (11) bzw. dem Hauptdeckel (2) zugewandte Seite mindestens ein Vorsprung (13) vorgesehen ist, der mit mindestens einer entsprechenden Vertiefung (12) in der Anschlagfuge (11) oder im Hauptdeckel (2) zusammenwirkt, um das endgültige Verschießen des Einfülldeckels (4) in der vertieften Öffnung (3) zu gewährleisten.

10. Behälter nach Anspruch 9, **dadurch gekennzeichnet, dass** die Vertiefung (12) aus einer Nute besteht, die in Bezug auf die Drehachse A des Einfülldeckels (4) in radialer Richtung verläuft.
